# EUROPÄISCHE PATENTSCHRIFT

(11) **EP 0 946 479 B1**
(45) Veröffentlichungstag und Bekanntmachung des Hinweises auf die Patenterteilung: **12.09.2001**
(21) Anmeldenummer: 97953759.4
(22) Anmeldetag: 09.12.1997
(51) Int. Cl.: C07C 45/38, C07C 45/39, C07C 47/127

(54) **VERFAHREN ZUR HERSTELLUNG VON CARBONYLVERBINDUNGEN**
METHOD FOR PRODUCING CARBONYL COMPOUNDS
PROCEDE DE PREPARATION DE COMPOSES CARBONYLE

(30) Priorität: 23.12.1996 DE 19654046
(43) Veröffentlichungstag der Anmeldung: 06.10.1999
(73) Patentinhaber: BASF AKTIENGESELLSCHAFT, 67056 Ludwigshafen (DE)
(72) Erfinder: FETZER, Thomas, D-67346 Speyer (DE); DEMUTH, Dirk, D-68161 Mannheim (DE); RÜTTER, Heinz, D-67126 Hochdorf-Assenheim (DE); MENIG, Helmuth, D-67159 Friedelsheim (DE); RESCH, Peter, D-67310 Hettenleidelheim (DE); RUPPEL, Wilhelm, D-67227 Frankenthal (DE); WACHE, Harro, D-67136 Fussgönheim (DE)
(86) Internationale Anmeldenummer: EP9706855
(87) Internationale Veröffentlichungsnummer: WO9828250

(56) Entgegenhaltungen:
- EP-A- 0 007 570
- EP-A- 0 271 812
- US-A- 4 555 583

## Beschreibung

Die Erfindung betrifft ein neues Verfahren zur Herstellung von Carbonylverbindungen durch Gasphasenoxidation von Alkoholen mit einem Sauerstoff enthaltenden Gas in Gegenwart von Kupfer und/ oder Silber enthaltenden Katalysatoren und einer unter den Reaktionsbedingungen flüchtigen Phosphorverbindung, die in zwei Anteilen zum einen dem gasförmigen Ausgangsgemisch zugesetzt und zum anderen direkt in die Katalysatorschüttung eingetragen wird.

Verfahren zur Herstellung von Carbonylverbindungen durch Gasphasenoxidation an Kupfer oder Silber-Katalysatoren in Gegenwart flüchtiger Phosphorverbindungen sind aus dem Stand der Technik bekannt.

So ist in der EP-A 007 570 ein Verfahren zur Herstellung von Glyoxal durch Gasphasenoxidation von Ethylenglykol mit Sauerstoff an einem Kupfer enthaltenden Oxidationskatalysator in Gegenwart von unter Reaktionsbedingungen flüchtigen Phosphorverbindungen beschrieben, bei dem die Phosphormenge 1 bis 100 ppm bezogen auf eingesetztes Ethylenglykol beträgt. Bei diesen Verfahren werden unbefriedigende Glyoxalausbeuten bis 70 Mol-%, bezogen auf umgesetztes Ethylenglykol, erzielt.

Nach den Verfahren der US-PS 4 282 374 und der US-PS 4 503 261 erhält man bei der Gasphasenoxidation von Ethylenglykol an Kupferkatalysatoren oder an einem Schichtkatalysator aus Kupfer- und Silberkristallen vorteilhafte Ergebnisse hinsichtlich der Lebensdauer der Katalysatoren und der Glyoxalausbeute, wenn man die Reaktion in Gegenwart einer flüchtigen Phosphorverbindung durchführt, wobei die Phosphormenge (berechnet P), bezogen auf die Gewichtsmenge an Ethylenglykol, 1 bis 100 ppm bzw. 0,5 bis 20 ppm beträgt. Bei diesen Verfahren hat sich jedoch bei längerer Betriebsdauer herausgestellt, daß die Glyoxalausbeute und Produktreinheit mit der Versuchsdauer zunehmend schlechter werden. Dieser Nachteil ist auf eine vermehrte Bildung von Formaldehyd und von CO/CO₂ zurückzuführen.

In der EP-B 0 271 812 wird für die Herstellung von Carbonylverbindungen wie Glyoxal, eine Gasphasenoxidation von Alkoholen mit einem Sauerstoff enthaltenden Gas in Gegenwart von Kupfer oder Silber enthaltenden Katalysatoren und einer unter den Reaktionsbedingungen flüchtigen Phosphorverbindung vorgeschlagen, bei der die Phosphorverbindung in einer Portion in einer Menge von weniger als 0,5 ppm, bezogen auf die Gewichtsmenge an eingesetztem Alkohol und berechnet als Phosphor, in dem gasförmigen Ausgangsgemisch vor der Umsetzung an dem Katalysator zugemischt wird. Nach dem in der EP-B 0 271 812 beschriebenen Verfahren wird Glyoxal in Ausbeuten bis 80 Mol-% erhalten.

Die vorstehenden Verfahren des Standes der Technik weisen den Nachteil einer unbefriedigenden Ausbeute auf. Bei dem bekannten Verfahren fällt Glyoxal als wäßrige, mit Glykolaldehyd, Formaldehyd und organischen Säuren verunreinigte Lösung an. Weitere unerwünschte Nebenprodukte sind die anfallenden Verbrennungsprodukte CO, CO₂ und H₂O. Als Folge der Nebenprodukte weisen die bekannten Verfahren zusätzlich den Nachteil unbefriedigender Katalysatorstandzeiten auf.

Anteile von Formaldehyd im Glyoxal sind für viele Anwendungszwecke des Glyoxals zudem wegen der toxikologischen Eigenschaften und der hohen Reaktivität des Formaldehyds höchst unerwünscht. Da man Formaldehyd nur mit einem erheblichen Aufwand und unter Inkaufnahme von Ausbeuteverlusten aus dem rohen Glyoxal entfernen kann, etwa durch Behandlung mit Wasserdampf oder durch chemische Umsetzung, war nach einem Verfahren zu suchen, das es gestattet, Glyoxal durch katalytische Gasphasenoxidation von Ethylenglykol auch bei langen Betriebszeiten unter weitgehender Vermeidung der Bildung störender Nebenprodukte herzustellen.

Es wurde nun gefunden, daß man bei der Herstellung von Carbonylverbindungen der Formel 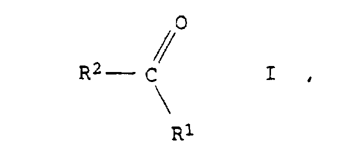 in der R¹ ein Wasserstoffatom oder einen Alkylrest mit 1 bis 3 C-Atomen, R² ein Wasserstoffatom oder einen Rest der Formel 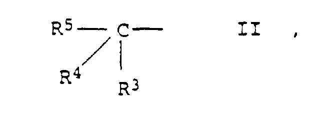 in der R³ ein Wasserstoffatom oder zusammen mit R⁴ ein Sauerstoffatom, R⁴ den Rest OR⁶ oder zusammen mit R³ ein Sauerstoffatom, R⁵ ein Wasserstoffatom, einen Alkylrest mit 1 bis 8 C-Atomen oder einen Cyclohexyl- oder Cyclopentylrest und R⁶ einen Alkylrest mit 1 bis 4 C-Atomen, einen Cyclohexyl- oder Cyclopentylrest oder einen Rest der Formel -CH₂-CHO oder -CH₂-CH₂-O-CH₂-CHO bedeuten, durch Gasphasenoxidation von Methanol oder Alkoholen der Formel 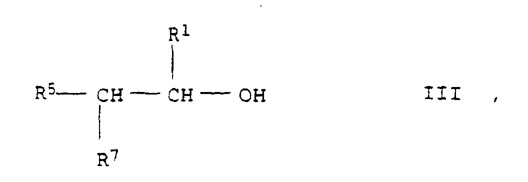 in der R¹ und R⁵ die oben angegebene Bedeutung haben und R⁷ ein Wasserstoffatom oder einen Rest OR⁸ und R⁸ ein Wasserstoffatom, einen Alkylrest mit 1 bis 4 C-Atomen, einen Cyclohexyl- oder Cyclopentylrest oder einen Rest der Formel -CH₂-CH₂-OH oder -CH₂-CH₂-O-CH₂-CH₂-OH bedeutet, mit einem Sauerstoff enthaltenden Gas in Gegenwart von Kupfer und/oder Silber enthaltenden Katalysatoren und einer unter Reaktionsbedingungen flüchtigen Phosphorverbindung in einer Menge, so daß die Phosphormenge (berechnet als P), bezogen auf die Gewichtsmenge an eingesetztem Alkohol, bis zu 20 ppm, bevorzugt 0,05 bis 20 ppm, beträgt, die Carbonylverbindungen auf besonders vorteilhafte Weise herstellen kann, wenn man die Phosphormenge in mindestens zwei Anteilen zugibt, wobei
a) der erste Anteil mit dem gasförmigen Ausgangsgemisch vor der Katalysatorschüttung und
b) mindestens ein weiterer Anteil innerhalb der Katalysatorschüttung, bevorzugt im Bereich der oberen 0,1 bis 50 % der Gesamthöhe der Katalysatorschüttung, besonders bevorzugt im Bereich der oberen 1 bis 35 % der Gesamthöhe der Schüttung, zugegeben wird.

Nach dem neuen Verfahren wird Glyoxal aus Ethylenglykol im Dauerbetrieb in hoher Ausbeute und Reinheit und bei deutlich reduziertem Nebenproduktanteil erhalten.

In den Alkoholen der Formel III bedeuten Alkylreste z.B. Methyl-, Ethyl-, Propyl- oder Butylreste. Bei dem erfindungsgemäßen Verfahren gehen die endständigen Hydroxylgruppen in Aldehydgruppen und die sekundären Hydroxylgruppen in Ketogruppen über.

Beispielsweise seien die folgende Ausgangsverbindungen der Formel III genannt:

HO ― CH₂ ― CH₂ ― OH , H₃COCH₂ ― CH₂OH , CH₃ ― CH₂OH ,

C₂H₅OCH₂ ― CH₂OH , H₃C ― CH(OH) ― CH₂ ― OH ,

C₂H₅ ― CH(OH) ― CH₂OH ,

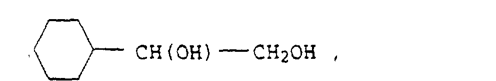

HO ― (CH₂)₂―O― (CH₂)₂―OH ,

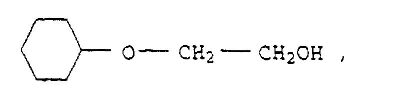

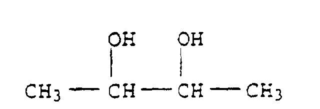

Die Gasphasenoxidation des Alkohols mit dem Sauerstoff enthaltenden Gas an den Kupfer und/oder Silber enthaltenden Katalysatoren führt man auf an sich bekannte Weise, z.B. bei Temperaturen von 225 bis 500°C, durch. Als Kupfer und/oder Silber enthaltende Katalysatoren sind beispielsweise metallisches Kupfer oder Silber, kupferhaltige oder silberhaltige Legierungen oder Verbindungen mit Metallen oder Nichtmetallen geeignet, wie Kupferphosphide, Kupferbronzen oder Legierungen des Kupfers mit Silber und/oder Gold, Kupfererze wie Malachit und Kupfer- oder Silberverbindungen, die während der Reaktion ganz oder teilweise zu Kupfer oder Silber reduziert werden können, wie Kupfer(I)oxid, Silber (I) oxid, Kupfer(II)oxid, und Verbindungen, die beim Erhitzen in Kupferoxide übergehen, wie Kupfernitrat und Kupferacetat. Ferner sind auch Kupferphosphat und Kupferantimonat geeignet. Den kupferhaltigen Verbindungen können zusätzlich noch andere Metall- oder Nichtmetalloxide wie die Oxide von Zink, Chrom, Phosphor, Antimon, Zinn und Wismut beigemischt sein. Die kupfer- und/oder silberhaltige katalytische Masse kann auch auf einem inerten Träger aufgebracht oder gegebenenfalls mit einem inerten Material verdünnt werden. Der Katalysator kann gegebenenfalls auch vor dem Einsatz einer reduzierenden Behandlung unterworfen werden.

Bevorzugt sind Katalysatoren, die keine große innere Oberfläche besitzen, beispielsweise solche mit einer Oberfläche von unter 50 m² pro g. Besonderes technisches Interesse haben metallisches Kupfer oder Silber und Legierungen, die Kupfer oder Silber als einen wesentlichen Bestandteil enthalten. Man wendet sie z.B. in Form von Drehspänen, Drahtgeweben, Gasen oder auch als Trägerkatalysatoren mit einem z.B. oberflächenarmen, inerten Träger an.

Als unter den Reaktionsbedingungen flüchtige Phosphorverbindungen verwendet man zweckmäßigerweise Phosphorverbindungen, die unzersetzt verdampfbar sind und mit den Komponenten des Synthesegases bei den Umsetzungsbedingungen keine Reaktion eingehen. Das sind z.B. Ester der Phosphorsäure, der phosphorigen Säure oder von Phosphonsäure, wie Trimethylphosphat, Triethylphosphat, Tri-isopropylphosphat, Tri-n-propylphosphat, Trimethylphosphit, Triethylphosphit, Triethylphosphinoxid, Methylphosphonsäurediethylester, Methylphosphonsäuredimethylester oder Ethylphosphonsäurediethylester.

Die Zugabe b) des zweiten oder gegebenenfalls weiterer Anteile der Phosphormenge erfolgt nach dem erfindungsgemäßen Verfahren innerhalb der Katalysatorschüttung, bevorzugt im Bereich der oberen 0,1 bis 50 % der Gesamthöhe der Katalysatorschüttung, besonders bevorzugt, im Bereich der oberen 1 bis 35 % der Katalysatorschüttung, nach dem Reaktorkopf. Die Zugabe b) innerhalb der Katalysatorschüttung erfolgt bevorzugt nach dem Hot Spot. Unter Hot Spot ist der Teil der Katalysatorschüttung zu verstehen, bei dem die höchste Temperatur innerhalb des Temperaturprofils der Katalysatorschüttung auftritt. Das Temperaturprofil der Katalysatorschüttung bzw. die Lage des Hot Spots wird üblicherweise bestimmt, indem man innerhalb der Katalysatorschüttung die Temperatur gegen die Schütthöhe bestimmt. Dies kann beispielsweise durch das Einbringen einer Thermohülse mit einem beweglichen Thermoelement oder aber durch ein feststehendes Multithermoelement mit mehreren Meßpunkten in Abhängigkeit von der Schütthöhe erfolgen.

Die Zugabe der Phosphormenge erfolgt bevorzugt in zwei Anteilen, bevorzugt von je 0,05 bis 10 ppm, insbesonders bevorzugt von je 0,1 bis 3 ppm.

Bei mehr als zwei Zugabestellen, beispielsweise drei Zugabestellen, wird die Gesamtmenge der flüchtigen Phosphormenge von 20 ppm bevorzugt in Anteile von 0,05 bis 10 ppm aufgeteilt.

Das Gewichtsverhältnis des ersten Anteils der Phosphormenge, der vor der Katalysatorschüttung zugegen wird, zu dem zweiten beziehungsweise der Summe der weiteren Anteile, die in die Katalysatorschüttung, bevorzugt nach dem Hot Spot, zugegeben werden, beträgt 0,005 bis 200, vorzugsweise 0,033 bis 30 und besonders bevorzugt 0,3 bis 3,3.

Das erfindungsgemäße Verfahren wird z.B. so ausgeführt, daß man ein gasförmiges Gemisch aus dem Alkohol und Wasser, wobei der Wassergehalt 0,1 bis 99 Gew.-% beträgt, zusammen mit Luft oder Sauerstoff in einer Menge zwischen 0,5 und 2,0 mol, bezogen auf 1 mol eingesetzten Alkohols, eventuell zusammen mit Stickstoff in einer Menge von bis zu 99 Vol.-% des Gesamtgasgemisches, über den auf 225 bis 500°C gehaltenen Katalysator leitet, wobei man den ersten Anteil an flüchtiger Phosphorverbindung dem gasförmigen Ausgangsgemisch zusetzt und mindestens einen weiteren Anteil in die Katalysatorschüttung nach dem Hot Spot im Bereich von 1 bis 35 % der Gesamthöhe der Schüttung zugibt.

Das den Reaktor verlassende Gasgemisch wird üblicherweise mit Wasser ausgewaschen.

Man kann die Phosphorverbindung als Lösung in Wasser, Alkohol, bevorzugt dem eingesetzten Alkohol, oder geeigneten Lösungsmitteln, wie zum Beispiel Ethern, in flüssiger oder durch Verdampfen der Lösung in gasförmiger Form oder aber in Form der reinen gasförmigen Phosphorverbindung zugeben, wobei die Zugabe als verdampfte Lösung oder in reiner gasförmiger Form bevorzugt ist.

Das nach dem erfindungsgemäßen Verfahren aus Ethylenglykol erhaltene Glyoxal, das direkt in der handelsüblichen Form einer 40 gew.-%igen wässrigen Lösung erhalten werden kann, zeichnet sich durch eine hohe Reinheit aus, die auch bei langem Betriebszeitraum unverändert erhalten bleibt. Durch das erfindungsgemäße Verfahren wird Glyoxal in hohen Ausbeuten bei langen Katalysatorstandzeiten erhalten.

Das erfindungsgemäße Verfahren wird anhand der nachfolgenden Beispiele näher erläutert.

### Beispiel 1

7,2 kg Kupferformkörper wurden in einem Rohrreaktor aus rostfreiem Stahl mit einem inneren Durchmesser von 55 mm so eingebaut, daß die Katalysatorfüllhöhe 250 cm beträgt (Katalysatorvolumen: 5,7 1). Man leitete pro Stunde ein Synthesegasgemisch bestehend aus 840 g Ethylenglykol, 1720 N1 Luft und 230 N1 Stickstoff durch den Rohrreaktor. Zum Synthesegas wurden vor der Katalysatorschüttung und bei 24 % der Gesamthöhe der Katalysatorschüttung nach dem Hot Spot, der bei 15 % der Gesamthöhe der Schüttung lag, jeweils 0,3 ppm P, bezogen auf die eingesetzte Gewichtsmenge an Ethylenglykol, in Form von Triethylphosphat gegeben. Die Reaktortemperatur wurde mit Hilfe einer Salzschmelze auf 365°C eingestellt.

Die Gesamtgasmenge, bestehend aus Kreisgas und Synthesegas, betrug 9150 Nl/h. Die GHSV (gas hourly space velocity), die definiert ist als

GHSV = Gasvolumen / Katalysatorvolumen

betrug 1610 h⁻¹. Die LHSV (liquid hourly space velocity), die definiert ist als

LHSV = Flüssigkeitsvolumen / Katalysatorvolumen

betrug 0,13 h⁻¹. Die Verweilzeit, definiert als Quotient aus dem Katalysatorvolumen und der Gasmenge war 2,3 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und CO₂ verblieben im Abgas und wurden in der Gasphase analysiert.

Nach einer Laufzeit von 10 Tagen erhielt man eine Glyoxalausbeute von 81,5 Mol-%, bezogen auf eingesetztes Ethylenglykol, bei einem Ethylenglykolumsatz von 99,6 Mol-%. Die Verbrennung zu CO und CO₂ betrug 11 Mol-%. Als weitere Nebenprodukte entstanden 0,5 Mol-% Glyoxaldehyd und 3,3 Mol-% Formaldehyd.

### Beispiel 2

Analog Beispiel 1 wurden 840 g/h Ethylenglykol, 1720 Nl/h Luft und 230 Nl/h Stickstoff durch den Rohrreaktor geleitet.

Zum Synthesegas wurden vor der Katalysatorschüttung 0,3 ppm P, bezogen auf die eingesetzte Gewichtsmenge an Ethylenglykol, und bei 24 % der Gesamthöhe der Katalysatorschüttung nach dem Hot Spot, der bei 15 % der Gesamthöhe der Schüttung lag, 0,6 ppm P, bezogen auf die eingesetzte Gewichtsmenge an Ethylenglykol, in Form von Triethylphosphat zugegeben.

GHSV, LHSV und Verweilzeit waren analog zu Beispiel 1. Man erhielt eine Glyoxalausbeute von 80,3 Mol-%, bezogen auf eingesetztes Ethylenglykol, bei einem Ethylenglykol-Umsatz von 99,4 Mol-%. Weiterhin erhielt man Glykolaldehyd mit 0,5 Mol-%, Formaldehyd mit 2,8 Mol-% und CO und CO₂ in einer Gesamtausbeute von 12 Mol-%.

### Beispiel 3

Die Reaktionsführung erfolgte analog zu Beispiel 2, mit dem Unterschied, daß die Luftmenge 1640 Nl/h und die Stickstoffmenge 290 Nl/h betrug. Man erhielt eine Glyoxalausbeute von 82,8 Mol-% bei einem Ethylenglykol-Umsatz von 98,4 Mol-%. Weiterhin erhielt man 1 Mol-% Glykolaldehyd, 1,9 Mol-% Formaldehyd und CO und CO₂ in einer Gesamtausbeute von 9 Mol-%, jeweils bezogen auf eingesetztes Ethylenglykol.

### Vergleichsbeispiel 1 (kein Zusatz flüchtiger P-Verbindung)

Man verfuhr wie in Beispiel 1 beschrieben. Es wurde jedoch keine flüchtige Phosphorverbindung zugesetzt. Die Produktaufarbeitung und die Gasanalytik erfolgte wie in Beispiel 1 beschrieben. Man erhielt eine Glyoxal-Ausbeute von 71,6 Mol-% bei einem Ethylenglykolumsatz von 94,3 Mol-%. Die Verbrennug zu CO und CO₂ betrug 13 Mol-%. Glykolaldehyd und Formaldehyd wurden 1,3 Mol-% und 6,1 Mol-% gebildet.

### Vergleichsbeispiel 2 (Zusatz flüchtiger P-Verbindungen zum Ausgangsgemisch)

Man verfuhr wie in Beispiel 1 beschrieben, wobei jedoch die Zugabe von Triethylphosphat ausschließlich vor der Katalysatorschüttung erfolgte. Dem Synthesegas wurden vor der Katalysatorschüttung 0,3 ppm P, bezogen auf die eingesetzte Gewichtsmenge an Ethylenglykol, in Form von Triethylphosphat zugegeben. Die Produktaufarbeitung und die Gasanalytik erfolgte wie in Beispiel 1 beschrieben. Man erhielt eine Glyoxalausbeute von 75,9 Mol-% bei einem Ethylenglykolumsatz von 98,9 Mol-%. Die Verbrennung zu CO und CO₂ betrug 14 Mol-%. Glykolaldehyd und Formaldehyd wurden 1,1 Mol-% und 5,9 Mol-% gebildet.

### Vergleichsbeispiel 3 (Zusatz flüchtiger P-Verbindung in die Katalysatorschüttung)

Man verfuhr wie in Beispiel 1 beschrieben, wobei jedoch die Zugabe von Triethylphosphat ausschließlich nach dem Hot Spot in die Katalysatorschüttung erfolgte. Bei 24 % der Gesamthöhe der Katalysatorschüttung wurden 0,3 ppm P, bezogen auf die eingesetzte Gewichtsmenge an Ethylenglykol, in Form von Triethylphosphat zum Synthesegas gegeben. Die Produktaufarbeitung und die Gasanalytik erfolgte wie in Beispiel 1 beschrieben. Man erhielt 76,9 Mol-% Glyoxal bei einem Ethylenglykol-Umsatz von 98,0 Mol - %.

Die Verbrennung zu CO und CO₂ betrug 13 Mol. Glykolaldehyd und Formaldehyd wurden 1,6 Mol-% und 5 Mol-% gebildet.

Die folgende Tabelle 1 stellt die mit den erfindungsgemäßen Beispielen 1 bis 3 den mit den nicht-erfindungsgemäßen Vergleichsbeispielen V1 bis V3 erzielten Umsätzen und Ausbeuten gegenüber.

**Tabelle 1:**

| Beispiel | Ethylenglykol-Umsatz [Mol-%] | Glyoxal-Ausbeute [Mol-%] | CO/CO₂ Ausbeute [Mol-%] | Glykolaldehyd Ausbeute [Mol%] | Formaldehyd Ausbeute [Mol-%] |
|---|---|---|---|---|---|
| 1 | 99,6 | 81,5 | 11 | 0,5 | 3,3 |
| 2 | 99,4 | 80,3 | 12 | 0,5 | 2,8 |
| 3 | 98,4 | 82,8 | 9 | 1,0 | 1,9 |
| V1 | 94,3 | 71,6 | 13 | 1,3 | 6,1 |
| V2 | 98,8 | 75,9 | 14 | 1,1 | 5,9 |
| V3 | 98,0 | 76,9 | 13 | 1,6 | 5,0 |

Tabelle 1 ist die mit dem erfindungsgemäßen Verfahren bei höheren Umsätzen erzielbare deutlich bessere Ausbeute an Glyoxal sowie der Rückgang der Ausbeuten der unerwünschten Nebenprodukte Glykolaldehyd und Formaldehyd zu entnehmen.

## Patentansprüche

1. Verfahren zur Herstellung von Carbonylverbindungen der Formel 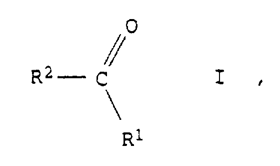 in der R¹ ein Wasserstoffatom oder einen Alkylrest mit 1 bis 3 C-Atomen, R² ein Wasserstoffatom oder einen Rest der Formel 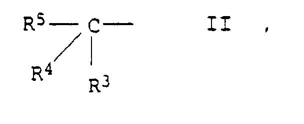 in der R³ ein Wasserstoffatom oder zusammen mit R⁴ ein Sauerstoffatom, R⁴ den Rest OR⁶ oder zusammen mit R³ ein Sauerstoffatom, R⁵ ein Wasserstoffatom, einen Alkylrest mit 1 bis 8 C-Atomen oder einen Cyclohexyl- oder Cyclopentylrest und R⁶ einen Alkylrest mit 1 bis 4 C-Atomen, einen Cyclohexyl- oder Cyclopentylrest oder einen Rest der Formel -CH₂-CHO oder -CH₂-CH₂-O-CH₂-CHO bedeuten, durch Gasphasenoxidation von Methanol oder Alkoholen der Formel 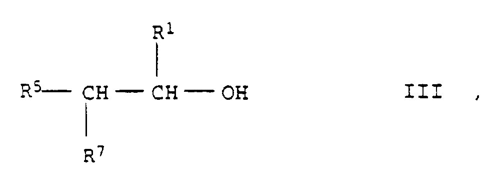 in der R¹ und R⁵ die oben angegebene Bedeutung haben und R⁷ ein Wasserstoffatom oder einen Rest OR⁸ und R⁸ ein Wasserstoffatom, einen Alkylrest mit 1 bis 4 C-Atomen, einen Cyclohexyl- oder Cyclopentylrest oder einen Rest der Formel -CH₂-CH₂-OH oder -CH₂-CH₂-O-CH₂-CH₂-OH bedeutet, mit einem Sauerstoff enthaltenden Gas in Gegenwart von Kupfer und/oder Silber enthaltenden Katalysatoren und einer unter Reaktionsbedingungen flüchtigen Phosphorverbindung in einer Menge, so daß die Phosphormenge (berechnet als P), bezogen auf die Gewichtsmenge an eingesetztem Alkohol, bis zu 20 ppm beträgt,
**dadurch gekennzeichnet, daß** die Phosphormenge in mindestens zwei Anteilen zugegeben wird, wobei
a) der erste Anteil mit dem gasförmigen Ausgangsgemisch vor der Katalysatorschüttung und
b) mindestens ein weiterer Anteil innerhalb der Katalysatorschüttung zugegeben wird.

2. Verfahren nach Anspruch 1, **dadurch gekennzeichnet, daß** mindestens ein weiterer Anteil b) in die Katalysatorschüttung im Bereich der oberen 0,1 bis 50 % der Gesamthöhe der Katalysatorschüttung zugegeben wird.

3. Verfahren nach Anspruch 1, **dadurch gekennzeichnet, daß** mindetens ein weiterer Anteil b) im Bereich der oberen 1 bis 35 % der Gesamthöhe der Katalysatorschüttung zugegeben wird.

4. Verfahren nach Anspruch 1, **dadurch gekennzeichnet, daß** die Phosphormenge in zwei Anteilen zugegeben wird.

5. Verfahren nach Anspruch 1, **dadurch gekennzeichnet, daß** die Phosphormenge (berechnet als P), bezogen auf die Gewichtsmenge an dem eingesetzten Alkohol, 0,05 bis 20 ppm beträgt.

6. Verfahren nach Anspruch 1, **dadurch gekennzeichnet, daß** das Gewichtsverhältnis des ersten Anteils der Phosphormenge zu dem mindestens einen weiteren Anteil 0,005 bis 200 beträgt.

7. Verfahren nach Anspruch 1, **dadurch gekennzeichnet, daß** man Glyoxal aus Ethylenglykol herstellt.

## Claims

1. A process for preparing carbonyl compounds of the formula 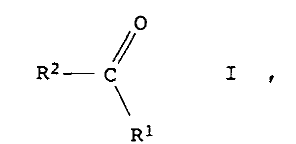 where R¹ is a hydrogen atom or an alkyl radical having from 1 to 3 carbon atoms, R² is a hydrogen atom or a radical of the formula 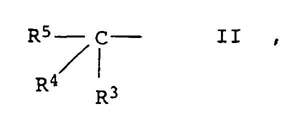 where R³ is a hydrogen atom or together with R⁴ is an oxygen atom, R⁴ is the radical OR⁶ or together with R³ is an oxygen atom, R⁵ is a hydrogen atom, an alkyl radical having from 1 to 8 carbon atoms or a cyclohexyl or cyclopentyl radical and R⁶ is an alkyl radical having from 1 to 4 carbon atoms, a cyclohexyl or cyclopentyl radical or a radical of the formula -CH₂-CHO or -CH₂-CH₂-O-CH₂-CHO, by gas-phase oxidation of methanol or alcohols of the formula 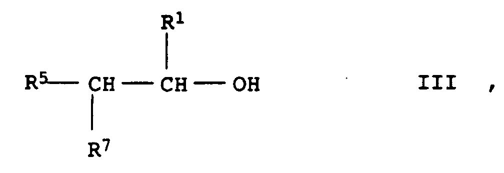 where R¹ and R⁵ are as defined above and R⁷ is a hydrogen atom or a radical OR⁸ and R⁸ is a hydrogen atom, an alkyl radical having from 1 to 4 carbon atoms, a cyclohexyl or cyclopentyl radical or a radical of the formula -CH₂-CH₂-OH or -CH₂-CH₂-O-CH₂-CH₂-OH, using an oxygen-containing gas in the presence of copper- and/or silver-containing catalysts and an amount of a phosphorus compound which is volatile under the reaction conditions which is such that the amount of phosphorus (calculated as P) is up to 20 ppm, based on the weight of alcohol used, wherein the phosphorus is introduced in at least two portions and
a) the first portion is introduced together with the gaseous starting mixture upstream of the catalyst bed, and
b) at least one further portion is introduced within the catalyst bed.

2. A process as claimed in claim 1, wherein at least one further portion b) is introduced into the catalyst bed in the region of the upper 0.1-50 % of the total height of the catalyst bed.

3. A process as claimed in claim 1, wherein at least one further portion b) is introduced in the region of the upper 1-35 % of the total height of the catalyst bed.

4. A process as claimed in claim 1, wherein the phosphorus is introduced in two portions.

5. A process as claimed in claim 1, wherein the amount of phosphorus (calculated as P) is from 0.05 to 20 ppm, based on the weight of the alcohol used.

6. A process as claimed in claim 1, wherein the weight ratio of the first portion of phosphorus to the further portion or portions is from 0.005 to 200.

7. A process as claimed in claim 1, wherein glyoxal is prepared from ethylene glycol.

## Revendications

1. Procédé de préparation de composés carbonyles de formule 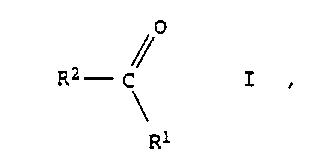 dans laquelle R¹ est un atome d'hydrogène ou un radical alkyle ayant de 1 à 3 atomes de carbone, R² est un atome d'hydrogène ou un radical de formule 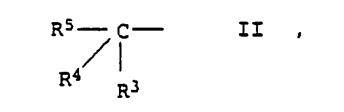 dans laquelle R³ est un atome d'hydrogène ou encore, avec R⁴, un atome d'oxygène, R⁴ est le radical OR⁶, ou encore avec R³ représente un atome d'oxygène, R⁵ est un atome d'hydrogène, un radical alkyle ayant de 1 à 8 atomes de carbone ou un radical cyclohexyle ou cyclopentyle, et R⁶ est un radical alkyle ayant de 1 à 4 atomes de carbone, un radical cyclohexyle ou cyclopentyle, ou un radical de formule -CH₂-CHO ou -CH₂-CH₂-O-CH₂-CHO, par oxydation en phase gazeuse de méthanol ou d'alcools de formule 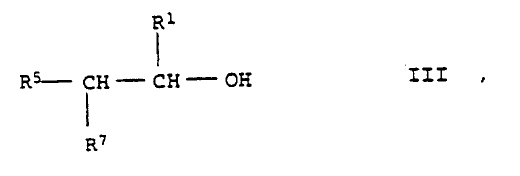 dans laquelle R¹ et R⁵ ont les significations données ci-dessus, et R⁷ est un atome d'hydrogène ou un radical OR⁸, et R⁸ est un atome d'hydrogène, un radical alkyle ayant de 1 à 4 atomes de carbone, un radical cyclohexyle ou cyclopentyle, ou un radical de formule -CH₂-CH₂-OH ou -CH₂-CH₂-O-CH₂-CH₂-OH, avec un gaz contenant de l'oxygène, en présence de catalyseurs contenant du cuivre et/ou de l'argent et d'un composé du phosphore, volatil dans les conditions de la réaction, en une quantité telle que la quantité de phosphore (calculée en P), par rapport au poids d'alcool utilisé, aille jusqu'à 20 ppm, **caractérisé en ce que** la quantité de phosphore est ajoutée en au moins deux portions, où
a) la première portion est ajoutée avec le mélange gazeux de départ, avant versement du catalyseur, et
b) au moins une deuxième portion est ajoutée en même temps que le versement du catalyseur.

2. Procédé selon la revendication 1, **caractérisé en ce qu'**au moins une autre portion b) est ajoutée à la masse de catalyseur versée, dans la zone correspondant aux 0,1 à 50 % supérieurs de la hauteur totale de la masse versée de catalyseur.

3. Procédé selon la revendication 1, **caractérisé en ce qu'**au moins une autre portion b) est ajoutée dans la zone des 1 à 35 % supérieurs de la hauteur totale de la masse versée de catalyseur.

4. Procédé selon la revendication 1, **caractérisé en ce que** la quantité de phosphore est ajoutée en deux portions.

5. Procédé selon la revendication 1, **caractérisé en ce que** la quantité de phosphore (calculée en P), est de 0,05 à 20 ppm par rapport au poids de l'alcool utilisé.

6. Procédé selon la revendication 1, **caractérisé en ce que** le rapport en poids de la première portion de la quantité de phosphore à au moins une autre portion est de 0,005 à 200.

7. Procédé selon la revendication 1, **caractérisé en ce qu'**on prépare du glyoxal à partir d'éthylèneglycol.
